Europäisches Patentamt

European Patent Office

Office européen des brevets

(11) Numéro de publication : **0 512 896 B1**

(12) **FASCICULE DE BREVET EUROPEEN**

(19)

⑪

(12)

(45) Date de publication du fascicule du brevet :
**28.09.94 Bulletin 94/39**

(51) Int. Cl.⁵ : **G01N 33/80**

(21) Numéro de dépôt : **92401221.4**

(22) Date de dépôt : **29.04.92**

(54) **Complexe agglutinant comme réactif de groupage sanguin.**

(30) Priorité : **02.05.91 FR 9105410**

(43) Date de publication de la demande :
**11.11.92 Bulletin 92/46**

(45) Mention de la délivrance du brevet :
**28.09.94 Bulletin 94/39**

(84) Etats contractants désignés :
**AT BE CH DE DK ES FR GB GR IT LI LU MC NL
PT SE**

(56) Documents cités :
**EP-A- 0 022 669
EP-A- 0 321 604
EP-A- 0 363 510**

(56) Documents cités :
**WO-A-90/07118
GB-A- 2 103 360
GB-A- 2 117 514
PATENT ABSTRACTS OF JAPAN, vol. 15, no.
119 (P-1183), 22 Mars 1991**

(73) Titulaire : **PASTEUR SANOFI DIAGNOSTICS
3 Boulevard Raymond Poincaré
F-92430 Marnes La Coquette (FR)**

(72) Inventeur : **Pernelle, Michel
74 Rue Gabriel Péri
F-91430 Igny (FR)**

(74) Mandataire : **Le Guen, Gérard et al
CABINET LAVOIX
2, place d'Estienne d'Orves
F-75441 Paris Cédex 09 (FR)**

EP 0 512 896 B1

## Description

L'invention a pour objet un complexe agglutinant utile pour la recherche des antigènes présents sur les érythrocytes, caractérisé en ce qu'il résulte de couplages d'affinité entre un anticorps de type IgG non agglutinant spécifique de l'antigène à identifier, une protéine capable de se lier en au moins deux sites à la partie Fc d'anticorps et un anticorps antiimmunoglobuline ou ses fragments.

La présente invention concerne un complexe agglutinant, et les réactifs qui le contiennent ainsi que leur utilisation pour l'identification des antigènes des érythrocytes, notamment des antigènes rares dans un test rapide d'agglutination.

Dans le domaine du groupage sanguin, un réactif, ou sérum-test, est dit agglutinant quand il est capable de produire en soluté salin une agglutination des cellules qui portent l'antigène spécifique de l'anticorps que contient le sérum-test; c'est le comportement le plus fréquemment observé des réactifs contenant des anticorps de type IgM; par contre, les anticorps de type IgG ne sont généralement pas agglutinants, même après un certain temps d'incubation.

Dans ce cas et, en particulier, pour les antigènes membranaires qui sont soit en faible nombre sur chaque cellule, soit peu accessibles aux anticorps, l'agglutination doit être provoquée au moyen d'artifices comme l'addition d'enzymes protéolytiques ou de fortes concentrations de protéines dans le milieu, ou encore en utilisant des IgG chimiquement modifiées comme dans WO 90/07118 et EP-A 022669.

Les exemples d'antigènes difficiles à mettre en évidence sont nombreux sur les cellules sanguines. Pour les globules rouges, on peut citer les antigènes des systèmes Rhésus (Rh), Lutheran, Kell (K), Duffy, Kidd, Diego et autres;

Il est souhaitable d'identifier ces antigènes parfois même en urgence, préalablement à une transfusion sanguine, au moins chez les polytransfusés, et préalablement à toute transplantation d'organe, ou encore lorsqu'il existe un risque d'alloimmunisation foeto-maternelle. Aussi, une technique rapide, simple de mise en oeuvre et peu coûteuse, comme celles permettant de constater directement l'existence d'une réaction immunologique grâce à l'apparition d'une agglutination, qui utilise un réactif agglutinant, spécifique, stable et de prix de revient faible, est d'un grand intérêt.

On sait qu'un groupage érythrocytaire peut être effectué par divers procédés simples et rapides qui peuvent même être automatisés, lorsque le réactif est agglutinant. On peut pour la description de ces procédés se référer à l'ouvrage de Rouger P. et Salmon C.: Techniques de Laboratoire 6 p.25-26 Ed. Masson (1981), qui décrit notamment les réactions sur plaque d'opaline, en tube ou en microplaque.

Mais, les réactifs utilisables dans ces techniques doivent être à base d'anticorps de type IgM, ceux de type IgG n'étant pas agglutinants. Aussi, on constate que les réactifs commercialisés actuellement concernent uniquement les groupages ABO ou Rhésus, systèmes pour lesquels les anticorps polyclonaux de type IgM sont naturellement abondants chez l'homme et, surtout, pour lesquels on peut obtenir des anticorps monoclonaux par culture d'hybridomes.

Par contre, l'obtention en quantité suffisante d'anticorps polyclonaux de type IgM pour satisfaire les besoins de la commercialisation d'un réactif de groupage sanguin dans le cas des antigènes difficiles à mettre en évidence, supposerait l'immunisation volontaire d'un grand nombre d'hommes pour permettre la récolte, dans les prélèvements de leur sang, des IgM spécifiques de l'antigène : celles-ci devraient, en outre, être ensuite séparées de toutes les autres IgM présentes avant leur utilisation. Or ces opérations coûteuses devraient être refaites indéfiniment de façon reproductible, puisque pour la plupart de ces antigènes, on ne sait pas actuellement préparer des anticorps monoclonaux de type IgM.

Un groupage érythrocytaire prenant en compte les antigènes pour lesquels ne sont disponibles en quantité suffisante que des anticorps de type IgG et donc des réactifs non agglutinants, ne peut être réalisé qu'avec des techniques plus complexes, décrites dans l'ouvrage précédent p.33-34 et 49 à 65; parmi elles, on peut citer les réactions sur plaque d'opaline, à 40°C, en présence de fortes concentrations de protéines ou autres polymères, dont l'albumine, les réactions en présence d'enzymes protéolytiques dont la papaïne ou les réactions dites à l'antiglobuline (test de Coombs) et leurs analogues.

On a aussi proposé des moyens pour obtenir un réactif agglutinant à partir d'anticorps de type IgG.

Par exemple, il a été exposé dans Proc. Natl. Acad. Scien. USA 74 (6) p. 2531 - 2535 (1977) que la réduction par le 2-mercapto-éthanol de l'IgG, le rendait agglutinant; mais ce procédé, au demeurant complexe car il implique une réaction chimique, ne donne pas le résultat attendu pour des antigènes peu flexibles ou présents sur un faible nombre de sites par cellule, comme l'antigène K; en outre, le procédé mis en oeuvre en tube nécessite une lecture au microscope après 2 heures d'incubation.

Dans GB-A-2103360, on décrit un réactif agglutinant composé d'anticorps IgG fixés, au niveau de leur région Fc, sur de la protéine A; mais l'agglutination n'est, là encore, pas immédiate; elle n'est observable, en tube, qu'après une incubation de 45 minutes à 37 ° C; en outre, l'aspect non homogène du réactif, préparé avec la bactérie entière, ne permet pas une utilisation aisée, notamment dans des appareils automatiques. Par contre, lorsque la protéine A est isolée de sa cellule bactérienne les antigènes rares comme le K, ne donne pas d'agglutination.

EP-A-0 321 604 décrit un complexe agglutinant comprenant deux molécules d'IgG et un anticorps anti-IgG pour l'identification des antigènes du système Rhésus.

La présente invention, selon un premier aspect, concerne un complexe agglutinant et les réactifs agglutinants le contenant, pour l'identification des antigènes érythrocytaires par des techniques d'agglutination simples et rapides, qui ne peuvent être effectuées qu'avec des anticorps de type IgM.

Le complexe selon l'invention peut évidemment être aussi utilisé dans les techniques classiques plus complexes, mais plus sensibles, dont l'emploi est parfois nécessaire pour confirmer un résultat douteux.

Le complexe agglutinant selon l'invention est le résultat de couplages d'affinité, dans une première étape, entre des anticorps de type IgG, spécifiques de l'antigène à identifier ou à doser, et des protéines capables de se lier en au moins deux points à la partie Fc des anticorps, puis dans une deuxième étape avec des anticorps anti-immunoglobuline.

Les anticorps de type IgG peuvent être monoclonaux ou polyclonaux.

Lorsque l'on utilise des anticorps monoclonaux, ils sont obtenus de façon classique, à partir de liquide d'ascites ou de surnageants, de préférence clarifiés, des cultures cellulaires d'hybridomes convenables; on trouve sur le marché des anticorps monoclonaux IgG, spéci-fiques de groupes sanguins difficiles à identifier; d'autres sont décrits dans la littérature. Les anticorps polyclonaux IgG peuvent être extraits des sérums ou plasmas de sujets humains immunisés, par exemple par le procédé d'absorption-élution décrit dans Vox. Sang. 33 p. 280 (1977); ils sont plus rares et plus coûteux.

Le complexe et les réactifs selon l'invention qui le contiennent sont particulièrement utiles pour l'identification des antigènes érythrocytaires difficiles à mettre en évidence et notamment ceux des systèmes Kell, Duffy ou Kidd pour lesquels sont rares les hommes spontanément immunisés et porteurs d'IgM et pour lesquels on ne connaît pas d'anticorps monoclonaux de type IgM de qualité suffisante pour permettre une reconnaissance des cellules sanguines.

Parmi les protéines capables de se fixer sur la partie Fc des anticorps et qui portent au moins deux sites de fixation pour former lors de la réaction immunologique avec l'antigène à déceler, la formation d'un réseau macromoléculaire, on peut citer celles d'origine microbiologique et notamment la protéine A de *Staphylococcus aureus*, bien connue, ou celles de *Staphylococcus epidermidis et de Staphylococcus pyogenes* ou encore, la protéine G de *Streptococcus species* ; ces protéines ont de nombreux sites de liaison.

Les protéines naturelles ou leurs fragments seront de préférence isolées des corps bactériens et purifiées, notamment pour avoir des lots reproductibles mais elles peuvent aussi être des protéines recombinantes; diverses protéines sont commercialisées notamment par les Sociétés SIGMA ou PIERCE, en général sous forme lyophilisée.

L'anticorps antiglobuline, autre constituant du complexe, peut être choisi parmi tous les types d'anticorps anti-immunoglobuline, polyvalents ou ceux spécifiques des IgG; il pourra être d'origine monoclonale ou polyclonale et plus ou moins purifié; des fragments, comportant les parties F(ab), ou tel que F(ab')$_2$ pourront aussi être utilisés. Lorsque l'anticorps IgG dirigé contre l'antigène à identifier est d'origine humaine, l'anticorps antiglobuline sera dirigé contre les immunoglobulines d'origine humaine; il importe en effet, d'avoir la même spécificité d'espèce pour les deux anticorps du complexe, à moins que les réactions d'affinité entre espèces soient possibles. Le complexe agglutinant selon l'invention peut être préparé par addition de la protéine, capable de se lier à la partie Fc des anticorps, à une solution de l'anticorp IgG dirigé contre l'antigène à détecter, en milieu aqueux de préférence tamponné à un pH compris entre 5 et 9, mieux entre, mieux entre 6,5 et 7,5, ou en milieu aqueux salin sensiblement isotonique, éventuellement tamponné; la réaction est terminée après un temps qui dépend notamment des concentrations et de la température d'incubation, généralement comprise entre 4°C et 50°C, de préférence entre 20°C et 40°C; dans cette dernière gamme de température, le temps nécessaire à la formation des liaisons est compris entre 5 mn et 1 heure.

La quantité de protéine ajoutée à la solution d'anticorps dépend de la concentration de ce dernier, mais aussi de la densité, sur la protéine, des sites de liaison de l'anticorps; il est préférable que les anticorps saturent sensiblement tous les sites. A titre d'exemple, on peut indiquer que le rapport de poids d'anticorps au poids de protéine A ou G pure sera en général compris entre 3 et 30, de préférence entre 5 et 20, et que la concentration en anticorps dans le milieu de liaison sera de préférence supérieure à 50 µg/ml.

L'addition de l'anti-IgG peut avoir lieu dès la fin de la première incubation, ou ultérieurement si le milieu est conservé à + 4° C, ou mieux, congelé. Comme dans la première étape, le volume et la concentration de la solution d'anticorps à ajouter dépendent notamment de la spécificité de l'anticorps et de la sensibilité recherchée. Par exemple, pour un groupage Kell ou Rhésus, on peut utiliser des quantités d'anticorps de type IgG et d'anti-immunoglobuline sensiblement identiques, pour former le complexe selon l'invention.

La température et la durée de la seconde incubation pour fixer l'anti-anticorps ne sont pas critiques; elles pourront évoluer dans les mêmes limites que celles indiquées pour la liaison protéine/premier anticorps. L'homme du métier sera à même de déterminer tous les paramètres de la préparation du complexe par des essais préablables.

On pourrait aussi, quoique cette méthode ne soit pas préférée, faire incuber un mélange des deux anticorps et de la protéine, en proportions convenables, pour obtenir le complexe agglutinant.

La solution de complexe agglutinant, obtenue à la fin des couplages d'affinité est généralement plus concentrée que celle qui sera utilisée dans les procédés d'identification rapides de routine, pour lesquels, la concentration en IgG complexée dans le réactif est, avantageusement de l'ordre de 1 à 50 µg/ml.

Aussi, en fin de préparation, elle peut être conservée à une température inférieure à 10° C et même congelée pour une dilution ultérieure extemporanée, ou elle peut être conservée diluée, prête à l'emploi, dans les mêmes conditions, après son conditionnement en multiples de doses unitaires. La dilution convenable est en général obtenue par addition au milieu de préparation du complexe d'une solution tampon de pH compris entre 5 et 9, à base de TRIS ou de phosphate, qui peut aussi contenir des sels hydrosolubles, des amino-acides ainsi que des macromolécules connues pour leur pouvoir de stabilisation des solutions d'anticorps et de renforcement des réactions d'agglutination mais à faible concentration, c'est-à-dire en général à concentration inférieure à 5 % ; à ces faibles concentrations il n'y a pas formation de faux positifs en présence d'IgG dans le sérum à tester. Parmi ces macromolécules, on peut citer la polyvinylpyrrolidone, les polysaccharides neutres, comme le Dextran, ou les protéines, comme l'albumine, bien connues des spécialistes.

Pour une conservation prolongée, on ajoutera aussi de préférence un agent bactériostatique, tel que l'azoture de sodium.

Ces réactifs et le complexe agglutinant de l'invention peuvent aussi être conservés lyophilisés ; ils sont alors introduits au moment de l'emploi dans de l'eau distillée ou dans un tampon de type phosphate salin, habituel dans ce domaine technique.

Selon un autre de ses aspects, l'invention concerne un procédé d'identification et de dosage des antigènes sur les globules rouges, qui consiste à mettre en présence les érythrocytes et le complexe agglutinant selon l'invention dans un milieu convenable et à observer s'il y a apparition d'une agglutination.

La composition du milieu dans lequel a lieu l'éventuelle agglutination n'est pas critique ; néanmoins, les cellules doivent y rester intactes, ce qui implique, en général, la présence de sels minéraux; par ailleurs, on a constaté que la présence de macromolécules, en quantité faible, inférieure à 5 %, améliorait la vitesse de la réaction; la nature de ces macromolécules et leur concentration dépend de la nature de l'anticorps, du complexe, de sa concentration et des conditions opératoires, mais l'homme du métier sera à même de les choisir après des essais préalables.

Ce procédé est avantageusement mis en oeuvre pour le groupage érythrocytaire et notamment pour la recherche de la présence des antigènes des systèmes Rh, Duffy, Kidd, ou Kell, pour lesquels ce procédé, simple et rapide, donne des résultats équivalents à la plupart des procédés utilisés actuellement dans les laboratoires, les hopitaux et les centres de transfusion sanguine.

On peut employer l'une quelconque des techniques rapides pour le groupage sanguin à base d,IgM, connues, pour effectuer la réaction immunologique et visualiser l'agglutination, que ce soit sur plaque d'opaline, en tube ou en microplaque. On peut opérer à une température comprise entre 10°C et 37°C, mais on préfère la température ambiante, plus simple de mise en oeuvre; l'agglutination est observée alors rapidement, en une minute à quelques minutes suivant les conditions opératoires, l'affinité de l'anticorps, la nature de l'antigène et sa concentration dans l'échantillon.

Dans ce qui suit, on décrit des exemples de complexes agglutinants et de réactifs de l'invention et les résultats de la mise en oeuvre du procédé selon l'invention d'identification d'antigènes rares, comparés à ceux obtenus avec des réactifs polyclonaux agglutinants riches en anticorps de type IgM.

Les différentes réactions d'agglutination utilisées dans les exemples ont été effectuées comme suit :

1°) Réaction sur plaque d'opaline à température ambiante :

Une goutte de réactif et une goutte du culot globulaire à tester, lavé ou non, sont déposées sur la plaque et mélangées avec une baguette de verre pour former un cercle de 2 cm de diamètre; l'agglutination éventuelle est constatée après 2 minutes.

2°) Réaction en tube :

Une goutte de suspension d'érythrocytes à 2 % et une goutte de réactif sont mélangées, et on observe les éventuels agglutinats formés après 1 ou 2 heures de sédimentation.

3°) Réaction à l'antiglobuline (ou test de Coombs):

On ajoute une goutte de suspension dans du sérum physiologique des globules rouges à tester, lavés ou non, dans un tube de Kahn contenant une goutte de réactif et on laisse incuber 15 minutes à 37°C avant de laver trois fois avec du sérum physiologique. Au culot de décantation, on ajoute 1 goutte d'antiglobuline et centrifuge durant 1 minute après la remise en suspension du culot; on observe ensuite l'agglutination éventuelle en agitant doucement le tube.

Les titres et scores des réactions ont été calculés de façon classique, comme il est décrit notamment dans l'ouvrage de Rouger P. et Salmon C., précédemment cité, p. 31-32.

EXEMPLE 1

Réactif anti-Kell agglutinant :
a) préparation :

On dilue par addition de tampon phosphate salin (pH 7,4) le surnageant clarifié d'une culture cellulaire contenant 150 µg/ml d'un anticorps anti-Kell monoclonal, d'origine humaine, de type IgG, dirigé contre l'antigène K, jusqu'à une concentration de l'anticorps de 100 µg/ml. A cette solution, on ajoute un volume d'une solution à 10 µg/ml dans le même tampon de protéine A, commmercialisée par Sigma sous la référence P 6031; le mélange est maintenu durant 60 minutes, sous agitation, à 37°C, puis on y ajoute 1 volume d'une solution d'antiglobuline polyvalente et on maintient le mélange durant 30 minutes, sous agitation, à 37°C avant de le ramener à température ambiante, vers 20°C.

L'antiglobuline polyvalente est un réactif préparé à partir de sérum de chèvre, hyperimmunisée avec des immunoglobulines et du complément humains, dilué dans un solvant salin isotonique contenant de l'albumine. Les caractéristiques de ce réactif répondent aux définitions du Journal Officiel de la République Française du 17 mars 1984, rubrique Ministère des Affaires Sociales - caractéristiques et normes des réactifs utilisés en immunohématologie érythrocytaire (Annexe 2 - p. 2596); ce réactif est commercialisé par Diagnostics Transfusion (FR) sous la référence 21120.

Pour les essais dont les résultats figurent dans ce qui suit, le mélange ainsi obtenu a été dilué avant utilisation avec le même volume de tampon phosphate salin (pH 7,4) contenant du glycocolle (2%) et du dextran (1%). Le réactif de référence, préparé à partir de sérums humains hyperimmuns, et enrichi en macromolécules, est commercialisé par Diagnostics Transfusion sous la référence 21083.
b) Tests d'agglutination :
1°) Réaction sur plaque d'opaline à température ambiante :
- sur 200 échantillons de sang de donneurs, dont 8% sont connus pour être du phénotype K$^+$, on a identifié 100% des phénotypes positifs, sans aucun taux positif ou négatif, avec le réactif selon l'invention et le réactif de référence. Au cours de cette étude, on a constaté que 45% des images d'agglutination obtenues avec le réactif selon l'invention étaient plus nettes et 50% équivalentes.
- la durée de formation des premiers agglutinats avec les deux réactifs était courte : 5 secondes pour le produit de cet exemple, 6 secondes pour la référence.
- les titres étaient respectivement de 8 et 4 et les scores de 20 et 14 pour le produit de cet exemple et pour la référence.

Si le réactif contient au lieu du complexe selon l'invention l'IgG utilisée pour sa préparation ou cette IgG liée à la seule protéine A, dans ces conditions aucune agglutination n'est observée.
2°) Réaction en tube et relecture au microscope optique :
Les résultats obtenus sont :

|  | Titre | Score |
|---|---|---|
| réactif de l'exemple 1 | 256 | 66 |
| réactif de référence | 128 | 56 |

3°) Réaction à l'antiglobuline :

| | Titre | Score |
|---|---|---|
| réactif de l'exemple 1 | 128 | 57 |
| réactif de référence | 256 | 71 |

EXEMPLE 2

Réactif anti-Kell agglutinant :

On prépare un réactif selon le procédé décrit à l'exemple 1 mais en remplaçant la protéine A par la protéine G, pure, commerciale.

Dans la réaction sur plaque d'opaline, le réactif a un titre de 8 et un score de 20 tandis qu'en tube son titre est de 128 et son score de 52 ; dans la réaction à l'antiglobuline, le titre est de 128 et le score de 56.

EXEMPLE 3

Réactif anti-Kell agglutinant :

On prépare un réactif selon le procédé décrit à l'exemple 1 avec la protéine A et une antiimmunoglobuline anti-IgG (H+L), dirigée contre les chaînes lourde et légère, d'origine humaine, commercialisée par Biosys France sous la référence BI 2015.

Sur plaque d'opaline, le titre est de 8 et le score de 20; en tube, le titre est de 128 et le score de 49; dans la réaction à l'antiglobuline, le titre est de 64 et le score de 46.

EXEMPLE 4

Réactif pour rechercher le phényotype D (Rh+)

Le réactif a été préparé avec un anticorps monoclonal de type IgG, dirigé contre l'antigène D, une antiimmunoglobuline humaine polyvalente et la protéine A en appliquant le procédé décrit à l'exemple 1.

Un réactif préparé dans les mêmes conditions avec uniquement l'anticorps monoclonal de départ dans la réaction sur plaque d'opaline ne donne pas d'agglutination, alors que le réactif du présent exemple a un titre de 16 et un score de 30; dans la réaction à l'antiglobuline plus complexe, l'anticorps monoclonal de départ a un score légèrement supérieur à celui du réactif du présent exemple.

**Revendications**

**Revendications pour les Etats contractants suivants : AT, BE, CH, DE, DK, FR, GB, GR, IT, LI, LU, MC, NL, SE, PT**

1.  Complexe agglutinant pour l'identification d'antigènes sur les érythrocytes, caractérisé en ce qu'il est constitué d'anticorps de type IgG non agglutinants spécifiques de l'antigène à identifier, sur lesquels on a fait réagir des protéines capables de se lier en au moins deux sites à la partie Fc des anticorps, puis des anticorps anti-immunoglobulines ou leurs fragments, les anticorps et les protéines étant liés par des liaisons d'affinité.

2.  Complexe selon la revendication 1, caractérisé en ce que les protéines sont choisies parmi les protéines A et G.

3.  Complexe selon l'une des revendications 1 et 2, caractérisé en ce que les deux types d'anticorps ont la même spécificité d'espèce.

4.  Complexe selon l'une des revendications 1 à 3, caractérisé en ce que l'anticorps anti-immunoglobuline est spécifique des IgG.

5.  Complexe selon l'une des revendications 1 à 4, caractérisé en ce que l'anticorps non agglutinant est un anticorps qui reconnaît les antigènes des systèmes Kell, Duffy ou Kidd.

6.  Complexe selon la revendication 5, caractérisé en ce que l'antigène est du système Kell.

7. Réactif agglutinant caractérisé en ce qu'il comprend un complexe selon l'une quelconque des revendications 1 à 6, à une concentration correspondant à 1 à 50 µg/ml d'IgG, dans une solution saline, tamponnée à un pH compris entre 5 et 9, et contenant de 0 à 5 % de macromolécules, utilisables dans les réactifs d'agglutination.

8. Réactif selon la revendication 7, caractérisé en ce qu'il contient des macromolécules choisies parmi la polyvinylpyrrolidone, les polysaccharides neutres et les protéines.

9. Procédé d'obtention d'un complexe selon l'une quelconque des revendications 1 à 6, caractérisé en ce que l'on met dans une première étape en présence, dans les conditions de formation des couplages d'affinité, l'anticorps non agglutinant spécifique de l'antigène à identifier et la protéine choisie, et que l'on met dans une seconde étape le produit couplé obtenu en présence de l'anticorps antiimmuglobuline dans les conditions de formation des couplages d'affinité.

10. Procédé d'identification des antigènes sur la paroi des érythrocytes, caractérisé en ce que l'on met en présence un complexe ou un réactif agglutinant selon l'une des revendications 1 à 8 avec les érythrocytes à caractériser et que l'on observe l'éventuelle agglutination.

11. Procédé d'identification d'antigène du système Kell dans un échantillon de sang, caractérisé en ce que l'on met cet échantillon en contact avec un complexe agglutinant selon la revendication 6 ou un réactif le contenant et que l'on observe l'éventuelle agglutination.

12. Procédé selon l'une des revendications 10 ou 11, caractérisé en ce qu'il est mis en oeuvre sur plaque.

**Revendications pour l'Etat contractant suivant : ES**

1. Procédé d'obtention d'un complexe agglutinant pour l'identification d'antigènes sur les érythrocytes constitué d'anticorps de type IgG non agglutinants spécifiques de l'antigène à identifier, sur lesquels on a fait réagir des protéines capables de se lier en au moins deux sites à la partie Fc des anticorps puis des anticorps anti-immunoglobulines ou leurs fragments liés par liaison d'affinité, caractérisé en ce que l'on met dans une première étape en présence, dans les conditions de formation des couplages d'affinité, les anticorps non agglutinants spécifiques de l'antigène à identifier et les protéines choisies et que l'on met dans une seconde étape le produit couplé obtenu en présence des anticorps anti-immunoglobulines ou leurs fragments dans les conditions de formation des couplages d'affinité.

2. Procédé selon la revendication 1, caractérisé en ce que les protéines sont choisies parmi les protéines A et G.

3. Procédé selon l'une des revendications 1 et 2, caractérisé en ce que les deux types d'anticorps ont la même spécificité d'espèce.

4. Procédé selon l'une des revendications 1 à 3, caractérisé en ce que l'anticorps anti-immunoglobuline est spécifique des IgG.

5. Procédé selon l'une des revendications 1 à 4, caractérisé en ce que l'anticorps non agglutinant est un anticorps qui reconnaît les antigènes des systèmes Kell, Duffy ou Kidd.

6. Procédé selon la revendication 5, caractérisé en ce que l'antigène est du système Kell.

7. Réactif agglutinant caractérisé en ce qu'il comprend un complexe agglutinant pour l'identification d'antigènes sur les érythrocytes constitué d'anticorps de type IgG non agglutinant spécifiques de l'antigène à identifier, de protéines capable de se lier en au moins deux sites à la partie Fc des anticorps et d'anticorps anti-immunoglobulines ou de ses fragments liés par liaison d'affinité, à une concentration correspondant à 1 à 50 ug/ml d'IgG, dans une solution saline, tamponnée à un pH compris entre 5 et 9, et contenant de 0 à 5 % de macromolécules, utilisables dans les réactifs d'agglutination.

8. Réactif selon la revendication 7, caractérisé en ce qu'il contient des macromolécules choisies parmi la polyvinylpyrrolidone, les polysaccharides neutres et les protéines.

9. Procédé d'identification des antigènes sur la paroi des érythrocytes, caractérisé en ce que l'on met en présence un complexe agglutinant pour l'identification d'antigènes sur les érythrocytes constitué d'anticorps de type IgG non agglutinants spécifiques de l'antigène à identifier, sur lesquels on a fait réagir des protéines capables de se lier en au moins deux sites à la partie Fc des anticorps puis des anticorps anti-immunoglobulines ou leurs fragments liés par liaison d'affinité, ou un réactif le contenant avec les érythrocytes à caractériser et que l'on observe l'éventuelle agglutination.

10. Procédé d'identification d'antigènes du système Kell dans un échantillon de sang, caractérisé en ce que l'on met cet échantillon en contact avec un complexe agglutinant pour l'identification d'antigènes sur les érythrocytes constitué d'anticorps de type IgG non agglutinants spécifiques de l'antigène à identifier, sur lesquels on a fait réagir des protéines capables de se lier en au moins deux sites à la partie Fc des anticorps puis des anticorpss anti-immunoglobulines ou leurs fragments liés par liaison d'affinité ou un réactif le contenant et que l'on observe l'éventuelle agglutination.

11. Procédé selon l'une des revendications 9 ou 10, caractérisé en ce qu'il est mis en oeuvre sur plaque.

## Patentansprüche

**Patentansprüche für folgende Vertragsstaaten : AT, BE, CH, DE, DK, FR, GB, GR, IT, LI, LU, MC, NL, PT, SE**

1. Agglutinierender Komplex zur Identifikation von Antigenen auf Erythrozyten, **dadurch gekennzeichnet**, daß er aus nicht-agglutinierenden Antikörpern des Typs IgG gebildet ist, die für das zu identifizierende Antigen spezifisch sind, welche man mit Proteinen, die in der Lage sind, sich an mindestens zwei Stellen des Fc-Teils der Antikörper zu binden, und dann mit Anti-Immunoglobulin-Antikörpern oder Fragmenten davon umgesetzt hat, wobei die Antikörper und die Proteine über Affinitätsbindungen gebunden sind.

2. Komplex nach Anspruch 1, **dadurch gekennzeichnet**, daß die Proteine aus Proteinen A und G ausgewählt sind.

3. Komplex nach einem der Ansprüche 1 und 2, **dadurch gekennzeichnet**, daß die beiden Antikörper-Typen die gleiche Art-Spezifität besitzen.

4. Komplex nach einem der Ansprüche 1 bis 3, **dadurch gekennzeichnet**, daß der Anti-Immunoglobulin-Antikörper für IgG spezifisch ist.

5. Komplex nach einem der Ansprüche 1 bis 4, **dadurch gekennzeichnet**, daß der nicht-agglutinierende Antikörper ein Antikörper ist, der Antigene der Systeme Kell, Duffy oder Kidd erkennt.

6. Komplex nach Anspruch 5, **dadurch gekennzeichnet**, daß das Antigen zum Kell-System gehört.

7. Agglutinierendes Reagens, **dadurch gekennzeichnet**, daß es einen Komplex nach einem der Ansprüche 1 bis 6 in einer 1 bis 50 μg/ml IgG entsprechenden Konzentraton in einer Salzlösung, die auf einen pH-Wert zwischen 5 und 9 gepuffert ist und 0 bis 5 % für Agglutinations-Reagenzien verwendbare Makromoleküle enthält, umfaßt.

8. Reagens nach Anspruch 7, **dadurch gekennzeichnet**, daß es aus Polyvinylpyrrolidon, neutralen Polysacchariden und Proteinen ausgewählte Makromoleküle enthält.

9. Verfahren zur Herstellung eines Komplexes nach einem der Ansprüche 1 bis 6, **dadurch gekennzeichnet**, daß man in einer ersten Stufe für das zu identifizierende Antigen spezifische nicht-agglutinierende Antikörper und das ausgewählte Protein unter Bedingungen der Bildung von Affinitäts-Kupplungen vereinigt und in einer zweiten Stufe das erhaltene gekuppelte Produkt mit Anti-Immunoglobulin-Antikörpern unter Bedingungen zur Bildung von Affinitäts-Kupplungen zusammenbringt.

10. Verfahren zur Identifikation von Antigenen auf der Wand von Erythrozyten, **dadurch gekennzeichnet**, daß man die zu identifizierenden Erythrozyten mit einem agglutinierenden Komplex oder Reagens nach einem der Ansprüche 1 bis 8 zusammenbringt und eine eventuelle Agglutination beobachtet.

11. Verfahren zur Identifikation von Antigen des Kell-Systems in einer Blutprobe, **dadurch gekennzeichnet**,

EP 0 512 896 B1

daß man die Probe mit einem agglutinierenden Komplex nach Anspruch 6 oder einem ihn enthaltenden Reagens in Kontakt bringt und eine eventuelle Agglutination beobachtet.

12. Verfahren nach einem der Ansprüche 10 oder 11, **dadurch gekennzeichnet**, daß es auf einem Plättchen durchgeführt wird.

**Patentansprüche für folgenden Vertragsstaat : ES**

1. Verfahren zur Herstellung eines agglutinierenden Komplexes zur Identifikation von Antigenen auf Erythrozyten, der aus nicht-agglutinierenden Antikörpern des Typs IgG gebildet ist, die für das zu identifizierende Antigen spezifisch sind, welche man mit Proteinen, die in der Lage sind, sich an mindestens zwei Stellen des Fc-Teils der Antikörper zu binden, und dann mit Anti-Immunoglobulin-Antikörpern oder Fragmenten davon umgesetzt hat, wobei die Antikörper und die Proteine über Affinitätsbindungen gebunden sind, **dadurch gekennzeichnet**, daß man in einer ersten Stufe für das zu identifizierende Antigen spezifische nicht-agglutinierende Antikörper und die ausgewählten Proteine unter Bedingungen der Bildung von Affinitäts-Kupplungen vereinigt und in einer zweiten Stufe das erhaltene gekuppelte Produkt mit Anti-Immunoglobulin-Antikörpern unter Bedingungen zur Bildung von Affinitäts-Kupplungen zusammenbringt.

2. Verfahren nach Anspruch 1, **dadurch gekennzeichnet**, daß die Proteine aus Proteinen A und G ausgewählt werden.

3. Verfahren nach einem der Ansprüche 1 und 2, **dadurch gekennzeichnet**, daß die beiden Typen von Antikörpern die gleiche Art-Spezifität besitzen.

4. Verfahren nach einem der Ansprüche 1 bis 3, **dadurch gekennzeichnet**, daß der Anti-Immunoglobulin-Antikörper für IgG spezifisch ist.

5. Verfahren nach einem der Ansprüche 1 bis 4, **dadurch gekennzeichnet**, daß der nicht-agglutinierende Antikörper ein Antikörper ist, der Antigene der Systeme Kell, Duffy oder Kidd erkennt.

6. Verfahren nach Anspruch 5, **dadurch gekennzeichnet**, daß das Antigen dem Kell-System angehört.

7. Agglutinierendes Reagens, **dadurch gekennzeichnet**, daß es einen agglutinierenden Komplex zur Identifikation von Antigenen auf Erythrozyten, der aus nicht-agglutinierenden Antikörpern des Typs IgG, die für das zu identifizierende Antigen spezifisch sind, Proteine, die in der Lage sind, sich an mindestens zwei Stellen des Fc-Teils der Antikörper zu binden, und Anti-Immunoglobulin-Antikörper oder Fragmente davon, die über Affinitätsbindungen gebunden sind, in einer 1 bis 50 µg/ml IgG entsprechenden Konzentration in einer auf einen pH-Wert zwischen 5 und 9 gepufferten Salzlösung, die 0 bis 5 % in Agglutinationsreagenzien verwendbare Makromoleküle enthält, umfaßt.

8. Reagens nach Anspruch 7, **dadurch gekennzeichnet**, daß es aus Polyvinylpyrrolidon, neutralen Polysacchariden und Proteinen ausgewählte Makromoleküle enthält.

9. Verfahren zur Identifikation von Antigenen auf der Wand von Erythrozyten, **dadurch gekennzeichnet**, daß man einen agglutinierenden Komplex zur Identifikation von Antigenen auf Erythrozyten, der aus nicht-agglutinierenden Antikörpern des Typs IgG gebildet ist, die für das zu identifizierende Antigen spezifisch sind, welche man mit Proteinen, die in der Lage sind, sich an mindestens zwei Stellen des Fc-Teils der Antikörper zu binden, und dann mit Anti-Immunoglobulin-Antikörpern oder Fragmenten davon umgesetzt hat, wobei die Antikörper und die Proteine über Affinitätsbindungen gebunden sind, oder ein diesen enthaltendes Reagens mit den zu charakterisierenden Erythrozyten in Kontakt bringt und eine eventuelle Agglutination beobachtet.

10. Verfahren zur Identifikation von Antigenen des Kell-Systems in einer Blutprobe, **dadurch gekennzeichnet**, daß man diese Probe mit einem agglutinierenden Komplex zur Identifikation von Antigenen auf Erythrozyten, der aus nichtagglutinierenden Antikörpern des Typs IgG gebildet ist, die für das zu identifizierende Antigen spezifisch sind, welche man mit Proteinen, die in der Lage sind, sich an mindestens zwei Stellen des Fc-Teils der Antikörper zu binden, und dann mit Anti-Immunoglobulin-Antikörpern oder Fragmenten davon umgesetzt hat, wobei die Antikörperund die Proteine über Affinitätsbindungen gebunden sind, oder einem ihn enthaltenden Reagens in Kontakt bringt und eine eventuelle Agglutination beobach-

9

tet.

11. Verfahren nach einem der Ansprüche 9 oder 10, **dadurch gekennzeichnet**, daß man es auf Plättchen durchführt.

## Claims

**Claims for the following Contracting States : AT, BE, CH, DE, DK, FR, GB, GR, IT, LI, LU, MC, NL, PT, SE**

1. Agglutinant complex for the identification of antigens on erythrocytes, characterised in that it consists of nonagglutinant IgG type antibodies which are specific for the antigen to be identified and which have been reacted with proteins capable of binding to at least two sites on the Fc part of the antibodies, as well as anti-immunoglobulin antibodies or fragments thereof, the antibodies and the proteins being bound through affinity bonding.

2. Complex according to claim 1, characterised in that the proteins are selected from proteins A and G.

3. Complex according to one of claims 1 and 2, characterised in that the two types of antibody have the same species specificity.

4. Complex according to one of claims 1 to 3, characterised in that the anti-immunoglobulin antibody is specific for IgG.

5. Complex according to one of claims 1 to 4, characterised in that the nonagglutinant antibody is an antibody which recognises the antigens of the Kell, Duffy or Kidd systems.

6. Complex according to claim 5, in which the antigen is of the Kell type.

7. Agglutinant reagent, characterised in that it comprises a complex according to any one of claims 1 to 6, at a concentration corresponding to 1 to 50 μg/ml of IgG in a saline solution buffered to a pH of between 5 and 9 and containing 0 to 5% of macromolecules which can be used in agglutination reagents.

8. Reagent according to claim 7, characterised in that it contains macromolecules chosen from polyvinylpyrrolidone, neutral polysaccharides and proteins.

9. Process for producing a complex according to any one or claims 1 to 6, characterised in that, in a first stage, the non agglutinant antibody specific for the antigen to be identified is contacted with the selected protein under conditions for forming affinity couplings, and in that, in a second stage, the coupled product obtained is contacted with the anti-immunoglobulin antibody under conditions for forming affinity couplings.

10. Process for the identification of antigens on the wall of erythrocytes, characterised in that an agglutinant complex or reagent according to one of claims 1 to 8 is contacted with the erythrocytes to be characterised and any agglutination which occurs is observed.

11. Process for the identification of a Kell type antigen in a blood sample, characterised in that this sample is contacted with an agglutinant complex according to claim 6 or a reagent containing it and any agglutination which occurs is observed.

12. Process according to one of claims 10 or 11, characterised in that it is carried out on plates.

**Claims for the following Contracting State : ES**

1. Process for producing an agglutinant complex for the identification of antigens on erythrocytes, consisting of nonagglutinant IgG type antibodies which are specific for the antigen to be identified and which have been reacted with proteins capable of binding to at least two sites on the Fc part of the antibodies, as well as anti-immunoglobulin antibodies or the fragments thereof bound through affinity bonding, characterised in that, in a first stage, the non agglutinant antibodies specific for the antigen to be identified is contacted with the selected proteins under conditions for forming affinity couplings, and in that, in a second stage, the coupled product obtained is contacted with the anti-immunoglobulin antibody under conditions for forming affinity couplings.

2. Process according to claim 1, characterised in that the proteins are selected from proteins A and G.

3. Process according to one of claims 1 and 2, characterised in that the two types of antibody have the same species specificity.

4. Process according to one of claims 1 to 3, characterised in that the anti-immunoglobulin antibody is specific for IgG.

5. Process according to one of claims 1 to 4, characterised in that the nonagglutinant antibody is an antibody which recognises the antigens of the Kell, Duffy or Kidd systems.

6. Process according to claim 5, characterised in that the antigen is of the Kell type.

7. Agglutinant reagent, characterised in that it comprises an agglutinant complex for the identification of antigens on erythrocytes, consisting of non-agglutinant IgG type antibodies specific for the antigen to be identified, proteins capable of binding to at least two sites on the Fc part of the antibodies, and anti-immunoglobulin antibodies or fragments thereof, linked by affinity bonding, at a concentration corresponding to 1 to 50 µg/ml of IgG in a saline solution buffered to a pH of between 5 and 9 and containing 0 to 5% of macromolecules which can be used in agglutination reagents.

8. Reagent according to claim 7, characterised in that it contains macromolecules chosen from polyvinylpyrrolidone, neutral polysaccharides and proteins.

9. Process for the identification of antigens on the wall of erythrocytes, characterised in that an agglutinant complex for the identification of antigens on erythrocytes, consisting of non-agglutinant, IgG-type antibodies specific for the antigen to be identified, which have been reacted with proteins capable of binding, at at least two sites, to the Fc part of the antibodies, as well as anti-immunoglobulin antibodies or the fragments thereof linked by affinity bonding, or a reagent containing said complex, is contacted with erythrocytes to be characterised and any agglutination which occurs is observed.

10. Process for the identification of Kell type antigens in a blood sample, characterised in that this sample is contacted with an agglutinant complex for the identification of antigens on erythrocytes, consisting of non-agglutinant, IgG-type antibodies specific for the antigen to be identified, which have been reacted with proteins capable of binding, at at least two sites, to the Fc part of the antibodies, as well as anti-immunoglobulin antibodies or the fragments thereof linked by affinity bonding, or a reagent containing said complex, and any agglutination which occurs is observed.

11. Process according to one of claims 9 or 10, characterised in that it is carried out on plates.